# EUROPEAN PATENT APPLICATION

(11) **EP 2 508 597 A1**
(43) Date of publication of application: **10.10.2012**
(21) Application number: 11161243.8
(22) Date of filing: 05.04.2011
(51) Int. Cl.: C12N 9/00, C12N 9/04, C12P 7/16

(54) **Production of butanol by fermentation in Arxula sp.**

(71) Applicant: Leibniz-Institut für Pflanzengenetik und Kulturpflanzenforschung (IPK), 06466 Gatersleben (DE); ACS Agrochemische Systeme GmbH, 66424 Homburg (DE); Jäckering Mühlen- und Nährmittelwerke GmbH, 59007 Hamm (DE)
(72) Inventor: Kunze, Gotthard, Prof. Dr., 06466 Gatersleben (DE); Hähnel, Urs, Dr., 06484 Quedlinburg (DE)
(74) Representative: Taruttis, Stefan Georg

(57) **Abstract**

The invention provides a non-conventional, non-pathogenic yeast, and a process using the yeast, for producing n-butanol. The yeast preferably is Arxula adeninivorans which has been genetically manipulated to contain a set of genes comprising the genes encoding the pathway enzymes for the conversion of acetyl-CoA to butyryl-CoA from a heterologous micro-organism, especially from Clostridium acetobutylicum.

SEQUENCE LISTING

<110>  Leibniz-Institut für Pflanzengenetik und
       Kulturpflanzenforschung
       ACS AGROCHEMISCHE SYSTEME GmbH
       Jäckering Mühlen- und Nährmittelwerke Gesellschaft mit
       beschränkter Haftung

<120>  Production of butanol by fermentation

<130>  I1009EP

<160>  18

<170>  PatentIn version 3.5

<210>  1
<211>  392
<212>  PRT
<213>  Clostridium acetobutylicum

<400>  1

Met Lys Glu Val Val Ile Ala Ser Ala Val Arg Thr Ala Ile Gly Ser
1               5                   10                  15

Tyr Gly Lys Ser Leu Lys Asp Val Pro Ala Val Asp Leu Gly Ala Thr
            20                  25                  30

Ala Ile Lys Glu Ala Val Lys Lys Ala Gly Ile Lys Pro Glu Asp Val
        35                  40                  45

Asn Glu Val Ile Leu Gly Asn Val Leu Gln Ala Gly Leu Gly Gln Asn
    50                  55                  60

Pro Ala Arg Gln Ala Ser Phe Lys Ala Gly Leu Pro Val Glu Ile Pro
65                  70                  75                  80

Ala Met Thr Ile Asn Lys Val Cys Gly Ser Gly Leu Arg Thr Val Ser
                85                  90                  95

Leu Ala Ala Gln Ile Ile Lys Ala Gly Asp Ala Asp Val Ile Ile Ala
            100                 105                 110

Gly Gly Met Glu Asn Met Ser Arg Ala Pro Tyr Leu Ala Asn Asn Ala
        115                 120                 125

Arg Trp Gly Tyr Arg Met Gly Asn Ala Lys Phe Val Asp Glu Met Ile
    130                 135                 140

Thr Asp Gly Leu Trp Asp Ala Phe Asn Asp Tyr His Met Gly Ile Thr
145                 150                 155                 160

Ala Glu Asn Ile Ala Glu Arg Trp Asn Ile Ser Arg Glu Glu Gln Asp
                165                 170                 175

Glu Phe Ala Leu Ala Ser Gln Lys Lys Ala Glu Glu Ala Ile Lys Ser
            180                 185                 190

Gly Gln Phe Lys Asp Glu Ile Val Pro Val Val Ile Lys Gly Arg Lys
        195                 200                 205

Gly Glu Thr Val Val Asp Thr Asp Glu His Pro Arg Phe Gly Ser Thr
    210                 215                 220

Ile Glu Gly Leu Ala Lys Leu Lys Pro Ala Phe Lys Lys Asp Gly Thr
225                 230                 235                 240

Val Thr Ala Gly Asn Ala Ser Gly Leu Asn Asp Cys Ala Ala Val Leu
                245                 250                 255

Val Ile Met Ser Ala Glu Lys Ala Lys Glu Leu Gly Val Lys Pro Leu
            260                 265                 270

Ala Lys Ile Val Ser Tyr Gly Ser Ala Gly Val Asp Pro Ala Ile Met
        275                 280                 285

Gly Tyr Gly Pro Phe Tyr Ala Thr Lys Ala Ala Ile Glu Lys Ala Gly
    290                 295                 300

Trp Thr Val Asp Glu Leu Asp Leu Ile Glu Ser Asn Glu Ala Phe Ala
305                 310                 315                 320

Ala Gln Ser Leu Ala Val Ala Lys Asp Leu Lys Phe Asp Met Asn Lys
                325                 330                 335

Val Asn Val Asn Gly Gly Ala Ile Ala Leu Gly His Pro Ile Gly Ala
            340                 345                 350

Ser Gly Ala Arg Ile Leu Val Thr Leu Val His Ala Met Gln Lys Arg
        355                 360                 365

Asp Ala Lys Lys Gly Leu Ala Thr Leu Cys Ile Gly Gly Gly Gln Gly
    370                 375                 380

Thr Ala Ile Leu Leu Glu Lys Cys
385                 390

<210>  2
<211>  282
<212>  PRT
<213>  Clostridium acetobutylicum

<400>  2

Met Lys Lys Val Cys Val Ile Gly Ala Gly Thr Met Gly Ser Gly Ile
1               5                   10                  15

Ala Gln Ala Phe Ala Ala Lys Gly Phe Glu Val Val Leu Arg Asp Ile
            20                  25                  30

Lys Asp Glu Phe Val Asp Arg Gly Leu Asp Phe Ile Asn Lys Asn Leu
        35                  40                  45

Ser Lys Leu Val Lys Lys Gly Lys Ile Glu Glu Ala Thr Lys Val Glu
    50                  55                  60

Ile Leu Thr Arg Ile Ser Gly Thr Val Asp Leu Asn Met Ala Ala Asp
65                  70                  75                  80

Cys Asp Leu Val Ile Glu Ala Ala Val Glu Arg Met Asp Ile Lys Lys
                85                  90                  95

Gln Ile Phe Ala Asp Leu Asp Asn Ile Cys Lys Pro Glu Thr Ile Leu
            100                 105                 110

Ala Ser Asn Thr Ser Ser Leu Ser Ile Thr Glu Val Ala Ser Ala Thr
        115                 120                 125

Lys Arg Pro Asp Lys Val Ile Gly Met His Phe Phe Asn Pro Ala Pro
    130                 135                 140

Val Met Lys Leu Val Glu Val Ile Arg Gly Ile Ala Thr Ser Gln Glu
145                 150                 155                 160

Thr Phe Asp Ala Val Lys Glu Thr Ser Ile Ala Ile Gly Lys Asp Pro
                165                 170                 175

Val Glu Val Ala Glu Ala Pro Gly Phe Val Val Asn Arg Ile Leu Ile
            180                 185                 190

Pro Met Ile Asn Glu Ala Val Gly Ile Leu Ala Glu Gly Ile Ala Ser
        195                 200                 205

Val Glu Asp Ile Asp Lys Ala Met Lys Leu Gly Ala Asn His Pro Met
    210                 215                 220

Gly Pro Leu Glu Leu Gly Asp Phe Ile Gly Leu Asp Ile Cys Leu Ala
225                 230                 235                 240

Ile Met Asp Val Leu Tyr Ser Glu Thr Gly Asp Ser Lys Tyr Arg Pro
                245                 250                 255

His Thr Leu Leu Lys Lys Tyr Val Arg Ala Gly Trp Leu Gly Arg Lys
            260                 265                 270

Ser Gly Lys Gly Phe Tyr Asp Tyr Ser Lys
        275                 280

<210> 3
<211> 261
<212> PRT
<213> Clostridium acetobutylicum

<400> 3

Met Glu Leu Asn Asn Val Ile Leu Glu Lys Glu Gly Lys Val Ala Val
1               5                   10                  15

Val Thr Ile Asn Arg Pro Lys Ala Leu Asn Ala Leu Asn Ser Asp Thr
            20                  25                  30

Leu Lys Glu Met Asp Tyr Val Ile Gly Glu Ile Glu Asn Asp Ser Glu
        35                  40                  45

Val Leu Ala Val Ile Leu Thr Gly Ala Gly Glu Lys Ser Phe Val Ala
    50                  55                  60

Gly Ala Asp Ile Ser Glu Met Lys Glu Met Asn Thr Ile Glu Gly Arg
65                  70                  75                  80

Lys Phe Gly Ile Leu Gly Asn Lys Val Phe Arg Arg Leu Glu Leu Leu
                85                  90                  95

Glu Lys Pro Val Ile Ala Ala Val Asn Gly Phe Ala Leu Gly Gly Gly
            100                 105                 110

Cys Glu Ile Ala Met Ser Cys Asp Ile Arg Ile Ala Ser Ser Asn Ala
        115                 120                 125

Arg Phe Gly Gln Pro Glu Val Gly Leu Gly Ile Thr Pro Gly Phe Gly
    130                 135                 140

Gly Thr Gln Arg Leu Ser Arg Leu Val Gly Met Gly Met Ala Lys Gln
145                 150                 155                 160

Leu Ile Phe Thr Ala Gln Asn Ile Lys Ala Asp Glu Ala Leu Arg Ile
                165                 170                 175

Gly Leu Val Asn Lys Val Val Glu Pro Ser Glu Leu Met Asn Thr Ala
            180                 185                 190

Lys Glu Ile Ala Asn Lys Ile Val Ser Asn Ala Pro Val Ala Val Lys
        195                 200                 205

Leu Ser Lys Gln Ala Ile Asn Arg Gly Met Gln Cys Asp Ile Asp Thr
    210                 215                 220

Ala Leu Ala Phe Glu Ser Glu Ala Phe Gly Glu Cys Phe Ser Thr Glu
225                 230                 235                 240

Asp Gln Lys Asp Ala Met Thr Ala Phe Ile Glu Lys Arg Lys Ile Glu
                245                 250                 255

Gly Phe Lys Asn Arg
            260

<210>  4
<211>  379
<212>  PRT
<213>  Clostridium acetobutylicum

<400>  4

Met Asp Phe Asn Leu Thr Arg Glu Gln Glu Leu Val Arg Gln Met Val
1               5                   10                  15

Arg Glu Phe Ala Glu Asn Glu Val Lys Pro Ile Ala Ala Glu Ile Asp
            20                  25                  30

Glu Thr Glu Arg Phe Pro Met Glu Asn Val Lys Lys Met Gly Gln Tyr
        35                  40                  45

Gly Met Met Gly Ile Pro Phe Ser Lys Glu Tyr Gly Gly Ala Gly Gly
    50                  55                  60

Asp Val Leu Ser Tyr Ile Ile Ala Val Glu Glu Leu Ser Lys Val Cys
65                  70                  75                  80

Gly Thr Thr Gly Val Ile Leu Ser Ala His Thr Ser Leu Cys Ala Ser
                85                  90                  95

Leu Ile Asn Glu His Gly Thr Glu Glu Gln Lys Gln Lys Tyr Leu Val
            100                 105                 110

Pro Leu Ala Lys Gly Glu Lys Ile Gly Ala Tyr Gly Leu Thr Glu Pro
        115                 120                 125

Asn Ala Gly Thr Asp Ser Gly Ala Gln Gln Thr Val Ala Val Leu Glu
    130                 135                 140

Gly Asp His Tyr Val Ile Asn Gly Ser Lys Ile Phe Ile Thr Asn Gly
145                 150                 155                 160

Gly Val Ala Asp Thr Phe Val Ile Phe Ala Met Thr Asp Arg Thr Lys
                165                 170                 175

Gly Thr Lys Gly Ile Ser Ala Phe Ile Ile Glu Lys Gly Phe Lys Gly
            180                 185                 190

Phe Ser Ile Gly Lys Val Glu Gln Lys Leu Gly Ile Arg Ala Ser Ser
        195                 200                 205

Thr Thr Glu Leu Val Phe Glu Asp Met Ile Val Pro Val Glu Asn Met
    210                 215                 220

Ile Gly Lys Glu Gly Lys Gly Phe Pro Ile Ala Met Lys Thr Leu Asp
225                 230                 235                 240

Gly Gly Arg Ile Gly Ile Ala Ala Gln Ala Leu Gly Ile Ala Glu Gly

245                 250                 255

Ala Phe Asn Glu Ala Arg Ala Tyr Met Lys Glu Arg Lys Gln Phe Gly
            260                 265                 270

Arg Ser Leu Asp Lys Phe Gln Gly Leu Ala Trp Met Met Ala Asp Met
        275                 280                 285

Asp Val Ala Ile Glu Ser Ala Arg Tyr Leu Val Tyr Lys Ala Ala Tyr
    290                 295                 300

Leu Lys Gln Ala Gly Leu Pro Tyr Thr Val Asp Ala Ala Arg Ala Lys
305                 310                 315                 320

Leu His Ala Ala Asn Val Ala Met Asp Val Thr Thr Lys Ala Val Gln
                325                 330                 335

Leu Phe Gly Gly Tyr Gly Tyr Thr Lys Asp Tyr Pro Val Glu Arg Met
            340                 345                 350

Met Arg Asp Ala Lys Ile Thr Glu Ile Tyr Glu Gly Thr Ser Glu Val
        355                 360                 365

Gln Lys Leu Val Ile Ser Gly Lys Ile Phe Arg
    370                 375

<210>  5
<211>  858
<212>  PRT
<213>  Clostridium acetobutylicum

<400>  5

Met Lys Val Thr Asn Gln Lys Glu Leu Lys Gln Lys Leu Asn Glu Leu
1               5                   10                  15

Arg Glu Ala Gln Lys Lys Phe Ala Thr Tyr Thr Gln Glu Gln Val Asp
            20                  25                  30

Lys Ile Phe Lys Gln Cys Ala Ile Ala Ala Ala Lys Glu Arg Ile Asn
        35                  40                  45

Leu Ala Lys Leu Ala Val Glu Glu Thr Gly Ile Gly Leu Val Glu Asp
    50                  55                  60

Lys Ile Ile Lys Asn His Phe Ala Ala Glu Tyr Ile Tyr Asn Lys Tyr
65                  70                  75                  80

Lys Asn Glu Lys Thr Cys Gly Ile Ile Asp His Asp Asp Ser Leu Gly
                85                  90                  95

Ile Thr Lys Val Ala Glu Pro Ile Gly Ile Val Ala Ala Ile Val Pro
            100                 105                 110

Thr Thr Asn Pro Thr Ser Thr Ala Ile Phe Lys Ser Leu Ile Ser Leu
        115                 120                 125

Lys Thr Arg Asn Ala Ile Phe Phe Ser Pro His Pro Arg Ala Lys Lys
    130                 135                 140

Ser Thr Ile Ala Ala Ala Lys Leu Ile Leu Asp Ala Ala Val Lys Ala
145                 150                 155                 160

Gly Ala Pro Lys Asn Ile Ile Gly Trp Ile Asp Glu Pro Ser Ile Glu
                165                 170                 175

Leu Ser Gln Asp Leu Met Ser Glu Ala Asp Ile Ile Leu Ala Thr Gly
            180                 185                 190

Gly Pro Ser Met Val Lys Ala Ala Tyr Ser Ser Gly Lys Pro Ala Ile
        195                 200                 205

Gly Val Gly Ala Gly Asn Thr Pro Ala Ile Ile Asp Glu Ser Ala Asp
    210                 215                 220

Ile Asp Met Ala Val Ser Ser Ile Ile Leu Ser Lys Thr Tyr Asp Asn
225                 230                 235                 240

Gly Val Ile Cys Ala Ser Glu Gln Ser Ile Leu Val Met Asn Ser Ile
                245                 250                 255

Tyr Glu Lys Val Lys Glu Glu Phe Val Lys Arg Gly Ser Tyr Ile Leu
            260                 265                 270

Asn Gln Asn Glu Ile Ala Lys Ile Lys Glu Thr Met Phe Lys Asn Gly
        275                 280                 285

Ala Ile Asn Ala Asp Ile Val Gly Lys Ser Ala Tyr Ile Ile Ala Lys
    290                 295                 300

Met Ala Gly Ile Glu Val Pro Gln Thr Thr Lys Ile Leu Ile Gly Glu
305                 310                 315                 320

Val Gln Ser Val Glu Lys Ser Glu Leu Phe Ser His Glu Lys Leu Ser
                325                 330                 335

Pro Val Leu Ala Met Tyr Lys Val Lys Asp Phe Asp Glu Ala Leu Lys
            340                 345                 350

Lys Ala Gln Arg Leu Ile Glu Leu Gly Gly Ser Gly His Thr Ser Ser
        355                 360                 365

Leu Tyr Ile Asp Ser Gln Asn Asn Lys Asp Lys Val Lys Glu Phe Gly
    370                 375                 380

Leu Ala Met Lys Thr Ser Arg Thr Phe Ile Asn Met Pro Ser Ser Gln
385                 390                 395                 400

Gly Ala Ser Gly Asp Leu Tyr Asn Phe Ala Ile Ala Pro Ser Phe Thr
                405                 410                 415

Leu Gly Cys Gly Thr Trp Gly Gly Asn Ser Val Ser Gln Asn Val Glu
            420                 425                 430

Pro Lys His Leu Leu Asn Ile Lys Ser Val Ala Glu Arg Arg Glu Asn
        435                 440                 445

Met Leu Trp Phe Lys Val Pro Gln Lys Ile Tyr Phe Lys Tyr Gly Cys
    450                 455                 460

Leu Arg Phe Ala Leu Lys Glu Leu Lys Asp Met Asn Lys Lys Arg Ala
465                 470                 475                 480

Phe Ile Val Thr Asp Lys Asp Leu Phe Lys Leu Gly Tyr Val Asn Lys
                485                 490                 495

Ile Thr Lys Val Leu Asp Glu Ile Asp Ile Lys Tyr Ser Ile Phe Thr
            500                 505                 510

Asp Ile Lys Ser Asp Pro Thr Ile Asp Ser Val Lys Lys Gly Ala Lys
        515                 520                 525

Glu Met Leu Asn Phe Glu Pro Asp Thr Ile Ile Ser Ile Gly Gly Gly
    530                 535                 540

Ser Pro Met Asp Ala Ala Lys Val Met His Leu Leu Tyr Glu Tyr Pro
545                 550                 555                 560

Glu Ala Glu Ile Glu Asn Leu Ala Ile Asn Phe Met Asp Ile Arg Lys
                565                 570                 575

Arg Ile Cys Asn Phe Pro Lys Leu Gly Thr Lys Ala Ile Ser Val Ala
            580                 585                 590

Ile Pro Thr Thr Ala Gly Thr Gly Ser Glu Ala Thr Pro Phe Ala Val
        595                 600                 605

Ile Thr Asn Asp Glu Thr Gly Met Lys Tyr Pro Leu Thr Ser Tyr Glu
    610                 615                 620

Leu Thr Pro Asn Met Ala Ile Ile Asp Thr Glu Leu Met Leu Asn Met
625                 630                 635                 640

Pro Arg Lys Leu Thr Ala Ala Thr Gly Ile Asp Ala Leu Val His Ala
                645                 650                 655

Ile Glu Ala Tyr Val Ser Val Met Ala Thr Asp Tyr Thr Asp Glu Leu

660                 665                 670

Ala Leu Arg Ala Ile Lys Met Ile Phe Lys Tyr Leu Pro Arg Ala Tyr
        675                 680                 685

Lys Asn Gly Thr Asn Asp Ile Glu Ala Arg Glu Lys Met Ala His Ala
    690                 695                 700

Ser Asn Ile Ala Gly Met Ala Phe Ala Asn Ala Phe Leu Gly Val Cys
705                 710                 715                 720

His Ser Met Ala His Lys Leu Gly Ala Met His His Val Pro His Gly
                725                 730                 735

Ile Ala Cys Ala Val Leu Ile Glu Glu Val Ile Lys Tyr Asn Ala Thr
            740                 745                 750

Asp Cys Pro Thr Lys Gln Thr Ala Phe Pro Gln Tyr Lys Ser Pro Asn
        755                 760                 765

Ala Lys Arg Lys Tyr Ala Glu Ile Ala Glu Tyr Leu Asn Leu Lys Gly
    770                 775                 780

Thr Ser Asp Thr Glu Lys Val Thr Ala Leu Ile Glu Ala Ile Ser Lys
785                 790                 795                 800

Leu Lys Ile Asp Leu Ser Ile Pro Gln Asn Ile Ser Ala Ala Gly Ile
                805                 810                 815

Asn Lys Lys Asp Phe Tyr Asn Thr Leu Asp Lys Met Ser Glu Leu Ala
            820                 825                 830

Phe Asp Asp Gln Cys Thr Thr Ala Asn Pro Arg Tyr Pro Leu Ile Ser
        835                 840                 845

Glu Leu Lys Asp Ile Tyr Ile Lys Ser Phe
    850                 855

<210>  6
<211>  1179
<212>  DNA
<213>  Artificial Sequence

<220>
<223>  /note="acetyl-CoA-acetyl transferase (THL), optimized codon
       usage, SEQ ID NO 6"

<400>  6
atgaaggagg tggtcattgc ttctgctgtg cgaactgcta ttggatctta cggaaagtct      60

ctgaaggacg tgcctgctgt ggacctggga gctactgcta ttaaggaggc tgtcaagaag     120

gctggtatta agcctgagga cgtgaacgag gtgattctgg gaaacgtgct gcaggctgga     180

ctgggacaga accctgctcg acaggcttct ttcaaggctg gactgcctgt cgagattcct     240

gctatgacta ttaacaaggt gtgcggatct ggactgcgaa ctgtgtctct ggctgctcag 300

attattaagg ctggtgacgc tgacgtgatt attgctggtg gaatggagaa catgtctcga 360

gccccttacc tggctaacaa cgctcgatgg ggataccgaa tgggaaacgc taagttcgtg 420

gacgagatga ttactgacgg actgtgggac gctttcaacg actaccacat gggaattact 480

gctgagaaca ttgctgagcg atggaacatt tctcgagagg agcaggacga gttcgctctg 540

gcttctcaga agaaggctga ggaggctatt aagtctggac agttcaagga cgagattgtg 600

cctgtggtga ttaagggacg aaagggtgag actgtggtgg acactgacga gcaccctcga 660

ttcggatcta ctattgaggg actggctaag ctgaagcctg ctttcaagaa ggacggaact 720

gttactgctg gaaacgcttc tggactgaac gactgcgctg ctgtgctggt gattatgtct 780

gctgagaagg ctaaggagct gggagtcaag cctctggcta agattgtgtc ttacggatct 840

gctggtgtgg accctgctat tatgggatac ggacctttct acgctactaa ggctgctatt 900

gagaaggctg gatggactgt ggacgagctg gacctgattg agtctaacga ggctttcgct 960

gctcagtctc tggctgtggc taaggacctg aagttcgaca tgaacaaggt gaacgtgaac 1020

ggtggtgcta ttgctctggg acaccctatt ggagcctctg gtgctcgaat tttggtgact 1080

ctggtgcacg ctatgcagaa gcgagacgct aagaagggac tggccactct gtgcattgga 1140

ggtggacagg gaactgctat tcttcttgag aagtgctaa 1179

<210> 7
<211> 849
<212> DNA
<213> Artificial Sequence

<220>
<223> /note="beta-hydroxy butyryl-CoA dehydrogenase (HBD), optimized
codon usage, SEQ ID NO 7"

<400> 7
atgaagaagg tgtgcgtcat tggagctgga actatgggat ctggaattgc tcaggctttc 60

gctgctaagg gattcgaggt ggtgctgcga gacattaagg acgagttcgt ggaccgagga 120

ctggacttca ttaacaagaa cctgtctaag ctggtgaaga agggaaagat tgaggaggct 180

actaaggtcg agattctgac tcgaatttct ggaactgtgg acctgaacat ggctgctgac 240

tgcgacctgg tgattgaggc tgctgtcgag cgaatggaca ttaagaagca gattttcgct 300

gacctggaca acatctgcaa gcctgagact attctggctt ctaacacttc ttctctgtct 360

attactgagg tggcttctgc tactaagact aacgacaagg tgattggaat gcacttcttc 420

aaccctgccc ctgtgatgaa gctggtcgag gtgattcgag gaattgctac ttctcaggag 480

actttcgacg ctgtcaagga gacttctatt gctattggaa aggaccctgt cgaggtggct 540

gaggctcctg gattcgtggt gaaccgaatt ttgattccta tgattaacga ggctgtggga 600

attttggctg agggaattgc ttctgtcgag gacattgaca aggctatgaa gctgggagct 660

aaccacccta tgggacctct tgagctggga gacttcattg gactggacat ttgcctggct 720

attatggacg tgctgtactc tgagactggt gactctaagt accgacctca cactctgctg 780

aagaagtacg tccgagctgg atggctggga cgaaagtctg gaaagggatt ctacgactac 840

tccaagtaa 849

<210> 8
<211> 786
<212> DNA
<213> Artificial Sequence

<220>
<223> /note="crotonase (CRT), optimized codon usage, SEQ ID NO 8"

<400> 8
atggagctga acaacgtgat tcttgagaag gagggaaagg tcgctgtcgt gactattaac 60

cgacctaagg ctctgaacgc cctgaactct gacactctga aggagatgga ctacgtcatt 120

ggagagattg agaacgactc tgaggtgctg gctgtgattc tgactggtgc tggtgagaag 180

tctttcgtgg ctggtgctga catttctgag atgaaggaga tgaacactat tgagggacga 240

aagttcggaa ttttgggaaa caaggtgttc cgacgacttg agctgcttga gaagcctgtg 300

attgctgctg tgaacggatt cgctctggga ggtggatgcg agattgctat gtcttgcgat 360

attcgaattg cttcttctaa cgctcgattc ggacagcctg aggtgggact gggaattact 420

cctggattcg gaggaactca gcgactgtct cgactggtgg gaatgggaat ggctaagcag 480

ctgattttca ctgctcagaa cattaaggct gacgaggctc tgcgaattgg actggtgaac 540

aaggtggtcg agccttctga gctgatgaac actgctaagg agattgctaa caagattgtg 600

tctaacgctc ctgtggctgt gaagctgtct aagcaggcta ttaaccgagg aatgcagtgc 660

gacattgaca ctgctctggc tttcgagtct gaggctttcg gagagtgctt ctctactgag 720

gaccagaagg acgctatgac tgctttcatt gagaagcgaa agattgaggg attcaagaac 780

cgataa 786

<210> 9
<211> 1140
<212> DNA
<213> Artificial Sequence

<220>
<223> /note="butyryl-CoA dehydrogenase (BDH), optimized codon usage,
SEQ ID NO 9"

<400> 9
atggacttca acctgactcg agagcaggag ctggtgcgac agatggtgcg agagttcgct 60

gagaacgagg tgaagcctat tgctgctgag attgacgaga ctgagcgatt ccctatggag 120

aacgtgaaga agatgggaca gtacggaatg atgggtattc cattctcgaa ggagtacggt 180

ggtgctggtg gtgacgtgct gtcttacatt attgctgtcg aggagctgtc taaggtgtgc 240

ggaactactg gtgtgattct gtctgctcac acttctctgt gcgcttctct gattaacgag 300

cacggaactg aggagcagaa gcagaagtac ctggtccctc tggctaaggg tgagaagatt 360

ggagcttacg gactgactga gcctaacgct ggaactgact ctggtgctca gcagactgtg 420

gctgtgcttg agggtgacca ctacgtgatt aacggatcta agattttcat cactaacggt 480

ggtgtggctg acactttcgt gattttcgct atgactgacc gaactaaggg tactaaggga 540

atttctgcct tcattattga gaagggattc aagggatttt ccattggaaa ggtcgagcag 600

aagctgggta ttcgagcttc ttctactact gagctggtgt tcgaggacat gattgtgcct 660

gtcgagaaca tgattggaaa ggagggaaag ggattcccta ttgctatgaa gactctggac 720

ggtggacgaa ttggaattgc tgctcaggct ctgggaattg ctgagggtgc tttcaacgag 780

gctcgagctt acatgaagga gcgaaagcag ttcggacgat ctctggacaa gttccaggga 840

ctggcttgga tgatggctga catggacgtg gctattgagt ctgctcgata cctggtgtac 900

aaggctgctt acctgaagca ggctggactg ccttacactg tggacgctgc tcgagctaag 960

ctgcacgctg ctaacgtggc tatggacgtg actactaagg ctgtgcagct gttcggagga 1020

tacggataca ctaaggacta ccctgtcgag cgaatgatgc gagatgctaa gattactgag 1080

atctacgagg gaacttctga ggtgcagaag ctggtcattt ctggaaagat tttccgataa 1140

<210> 10
<211> 2577
<212> DNA
<213> Artificial Sequence

<220>
<223> /note="butyraldehyde dehydrogenase (ADHE2), optimized codon
 usage, SEQ ID NO 10"

<400> 10
atgaaggtca ctaaccagaa ggagctgaag cagaagctga acgagctgcg agaggctcag 60

aagaagttcg ctacttacac tcaggagcag gtcgataaga ttttcaagca gtgcgctatt 120

gctgctgcta aggagcgaat caacctggct aagctggctg tcgaggagac tggaattgga 180

ctggtcgagg acaagattat taagaaccac ttcgctgctg agtacatcta caacaagtac 240

aagaacgaga agacctgcgg aattattgac cacgacgact ccctgggaat tactaaggtc 300

gctgagccta ttggaattgt cgctgctatt gtccctacta ccaaccctac ttctactgct 360

attttcaagt ctctgatttc ccttaagacc cgaaacgcta ttttcttctc gcctcaccct 420

cgagctaaga agtctactat tgctgccgcc aagctgattc tggacgctgc tgtcaaggct 480

ggtgctccta agaacattat tggatggatt gacgagcctt ctattgagct gtctcaggac 540

ctgatgtctg aggctgacat tattctggct actggtggac cttctatggt caaggctgct 600

tactcttctg gaaagcctgc tattggagtc ggagctggaa acactcctgc tattattgac 660

gagtctgctg acattgacat ggctgtctct tctattattc tgtccaagac ttacgacaac 720

ggtgtcattt gcgcttccga gcagtctatt ctggtcatga actctatcta cgagaaggtc 780

aaggaggagt tcgtcaagcg aggatcttac attctgaacc agaacgagat tgctaagatc 840

aaggagacta tgttcaagaa cggtgctatt aacgctgaca ttgtcggaaa gtctgcttac 900

attattgcta agatggctgg aattgaggtc cctcagacta ctaagattct gattggagag 960

gtccagtctg tcgagaagtc tgagctgttc tctcacgaga agctgtctcc cgtcctggct 1020

atgtacaagg tcaaggactt cgacgaggct ctgaagaagg ctcagcgatt gattgagctg 1080

ggaggatctg gacacacttc ttctctgtac attgactctc agaacaacaa ggacaaggtg 1140

aaggagttcg gactggctat gaagacttct cgaaccttca ttaacatgcc ttcgtcccag 1200

ggtgcttctg gtgacctgta caacttcgct attgctcctt ctttcactct gggatgcgga 1260

acttggggag gaaactctgt ctctcagaac gtcgagccta agcaccttct gaacattaag 1320

tctgtcgctg agcgacgaga gaacatgctg tggttcaagg tgcctcagaa gatctacttc 1380

aagtacggat gcctgcgatt cgctctgaag gagcttaagg acatgaacaa gaagcgagct 1440

ttcattgtca ctgacaagga cctgttcaag ctgggatacg tcaacaagat tactaaggtt 1500

ctggacgaga ttgacatcaa gtactctatt ttcaccgaca ttaagtccga ccctactatt 1560

gactctgtca agaagggtgc taaggagatg ctgaacttcg agcctgacac tattatttct 1620

attggaggtg gatctcctat ggacgctgct aaggtcatgc acctgttgta cgagtaccct 1680

gaggctgaga ttgagaacct ggctattaac ttcatggaca ttcgaaagcg aatctgcaac 1740

ttccctaagc tgggaactaa ggctatttct gtcgctattc ctactactgc tggaactgga 1800

tctgaggcta ctcctttcgc tgtcattacc aacgacgaga ctggaatgaa gtaccctctg 1860

acttcttacg agctgactcc taacatggct attattgata ctgagctgat gctgaacatg 1920

cctcgaaagc tgactgctgc tactggaatt gacgctctgg tccacgctat tgaggcttac 1980

gtgtctgtca tggctactga ctacactgac gagctggctc tgcgagctat taagatgatt 2040

ttcaagtacc tgcctcgagc ttacaagaac ggaactaacg acattgaggc tcgagagaag 2100

atggctcacg cttctaacat tgctggaatg gctttcgcta acgctttcct gggagtctgc 2160

cactctatgg ctcacaagct gggagctatg caccacgtcc ctcacggaat tgcttgcgct 2220

gtcctgattg aggaggtcat taagtacaac gctactgact gccctactaa gcagactgca 2280

ttccctcagt acaagtctcc taacgctaag cgaaagtacg ctgagattgc tgagtacctg 2340

aacctgaagg gaacttctga cactgagaag gtcactgctc tgattgaggc tatttctaag 2400

ctgaagattg acctgtctat tccccagaac atttctgctg ctggtattaa caagaaggac 2460

ttctacaaca ctctggacaa gatgtctgag ctggctttcg acgaccagtg cactactgct 2520

aaccctcgat accctctgat ttctgagctg aaggacatct acattaagtc cttctag 2577

<210> 11
<211> 885
<212> DNA
<213> Artificial Sequence

<220>
<223> /note="promoter element AYNR1, SEQ ID NO 11"

<400> 11
agcagtgatt tagcagcttc tagtcccagc agaccaccgc cgacgacaat ggcgcgcttt 60

cctttagtct caaacttgtc ggtagaaaag cgaatcattt cctgaagatc atccactgtg 120

cgatagacaa aaattccatg cgaacgagat tcctccacgg atggctcgta tccaagggac 180

cgagagatga tgtccgatgg tagaactgcc cggcttcctg tagccatgat taggagatca 240

tagctatatt cgtgccccaa gttagtcaaa accttcttgg tcttagtatc aatgctagtg 300

acgggctcgt taactcggca caccatatgg tgtggatcta tgctttcata ccaggcagtg 360

tcattgagat agaggtcatt gactgactgg tgcgaaaagt agtcggccaa tcccactcgg 420

ttgtaggcca aatacttctc ttctcctaga atggtaacgg aatattctct ggttaataga 480

tccctttcca gaagctgttc cacaaacgac atgccaacca ttccaaagcc caccaggaca 540

atttgctttg gcggagtggc caatccgttg tgtgttattg tcatggatcc tgtaagtgtg 600

tagtcaagca attgcaaaac gttgaccagg atgatccaag taataaatta ctttaccctt 660

atcatgctta tcgtccactc gggtggcctc ggggtttttc gaaccgaact gataagatag 720

tgccccattt tcgggacgaa agacgccgat ccgagataag acccctcgga acatggtgat 780

aagtaatcct cgcaaataga ttggacagtt tgacaatgat tagagttttg gagaatatat 840

agacgttatc agtacatggg gggggatagt tggcattgac tattg 885

<210> 12
<211> 465
<212> DNA
<213> Artificial Sequence

<220>
<223> /note="promoter element AYNR1´, SEQ ID NO 12"

<400> 12
ttgtaggcca aatacttctc ttctcctaga atggtaacgg aatattctct ggttaataga 60

tccctttcca gaagctgttc cacaaacgac atgccaacca ttccaaagcc caccaggaca 120

atttgctttg gcggagtggc caatccgttg tgtgttattg tcatggatcc tgtaagtgtg 180

tagtcaagca attgcaaaac gttgaccagg atgatccaag taataaatta ctttaccctt 240

atcatgctta tcgtccactc gggtggcctc ggggtttttc gaaccgaact gataagatag 300

tgccccattt tcgggacgaa agacgccgat ccgagataag acccctcgga acatggtgat 360

aagtaatcct cgcaaataga ttggacagtt tgacaatgat tagagttttg gagaatatat 420

agacgttatc agtacatggg gggggatagt tggcattgac tattg 465

<210> 13
<211> 599
<212> DNA
<213> Artificial Sequence

<220>
<223> /note="promoter element AYNI1, SEQ ID NO 13"

<400> 13
tctcaaccat gccatcaatc tcaatctccc agtccagcac gtcgttcgta gattcaggta 60

ccggtccatg gttacggaca aagtggagcg acgagggtgt aatgaatcca gcgttgtaca 120

acaagtccaa aggtgcctca gaattaaacg gatgagctcc agtcaacctc acgagccgtt 180

cgtcccgagg aacatgcgag tctggtgtct tcttgtcgag ttccagcaca tgttcagctt 240

tggacggtcc ttgaaacgct ggcccggtca gaattttttc aactgtttgg tattctggtg 300

ccatcaatag tcaatgccaa ctatcccccc ccatgtactg ataacgtcta tatattctcc 360

aaaactctaa tcattgtcaa actgtccaat ctatttgcga ggattactta tcaccatgtt 420

ccgaggggtc ttatctcgga tcggcgtctt tcgtcccgaa aatggggcac tatcttatca 480

gttcggttcg aaaaaccccg aggccacccg agtggacgat aagcatgata agggtaaagt 540

aatttattac ttggatcatc ctggtcaacg ttttgcaatt gcttgactac acacttaca 599

<210> 14
<211> 299
<212> DNA
<213> Artificial Sequence

<220>
<223> /note="promoter element AYNI1´, SEQ ID NO 14"

<400> 14
ccatcaatag tcaatgccaa ctatcccccc ccatgtactg ataacgtcta tatattctcc 60

aaaactctaa tcattgtcaa actgtccaat ctatttgcga ggattactta tcaccatgtt 120

ccgaggggtc ttatctcgga tcggcgtctt tcgtcccgaa aatggggcac tatcttatca 180

gttcggttcg aaaaaccccg aggccacccg agtggacgat aagcatgata agggtaaagt 240

aatttattac ttggatcatc ctggtcaacg ttttgcaatt gcttgactac acacttaca 299

<210> 15
<211> 663
<212> DNA
<213> Artificial Sequence

<220>
<223> /note="promoter element AYNT1, SEQ ID NO 15"

<400> 15
cttcggaaac agatatgagg atgatatttt atgcaaaagc gaattgtgca gatttgaaca 60

aatgacgcag gggcggatcc ggataaagct ctggctatct ggcaaaatgg accaatgcaa 120

tactcagcat actcccggaa ggatcgatct gcaaataatg gatcgtgagc tgtcgcacat 180

gaaggagggt cacgattcaa tgctgctagt atgtggacca gaaggaatgg tggagactgc 240

taaaacatgg caaaagtcca ggaacattga agattttagg gtagtctatt tttaagcata 300

taacagatat tttaagaaaa ttggcatagc tctacttcac ttttgtaacg acagctcgga 360

tcctggtaat accgagtttt aaagaaaaat gaattgttca catggaccat gcagtatctg 420

ataaagcata gataacgtta agtgagtgcg caccgtcctt aacctcggtt cgggtcaatg 480

aaaaccgagc gaaagcggaa ttttcagacc gagacaccct tatcaggtcg ttatcagaac 540

cttatctcga tcttatcagc tataacgctc agcggggtcg tatgcaaggg acaggtttaa 600

ataatgtctc ggaattggca tataataaat accgttgaaa ctttcataac tacagcatag 660

aca 663

<210> 16
<211> 331
<212> DNA
<213> Artificial Sequence

<220>

<223>  /note="promoter element AYNT1´, SEQ ID NO 16"

<400>  16
tacttcactt ttgtaacgac agctcggatc ctaccgagtt ttaaagaaaa atgaattgtt      60

cacatggacc atgcagtatc tgataaagca ttggtaaaga taacgttaag tgagtgcgca     120

ccgtccttaa cctcggttcg ggtcaatgaa aaccgagcga aagcggaatt ttcagaccga     180

gacaccctta tcaggtcgtt atcagaacct tatctcgatc ttatcagcta taacgctcag     240

cggggtcgta tgcaagggac aggtttaaat aatgtctcgg aattggcata taataaatac     300

cgttgaaact ttcataacta cagcatagac a                                    331


<210>  17
<211>  839
<212>  DNA
<213>  Artificial Sequence

<220>
<223>  /note="promoter element AYNR1-2, SEQ ID NO 17"

<400>  17
agcagtgatt tagcagcttc tagtcccagc agaccaccgc cgacgacaat ggcgcgcttt      60

cctttagtct caaacttgtc ggtagaaaag cgaatcattt cctgaagatc atccactgtg     120

cgatagacaa aaattccatg cgaacgagat tcctccacgg atggctcgta tccaagggac     180

cgagagatga tgtccgatgg tagaactgcc cggcttcctg tagccatgat taggagatca     240

tagctatatt cgtgccccaa gttagtcaaa accttcttgg tcttagtatc aatgctagtg     300

acgggctcgt taactcggca caccatatgg tgtggatcta tgctttcata ccaggcagtg     360

tcattgagat agaggtcatt gactgactgg tgcgaaaagt agtcggccaa tcccactcgg     420

ttgtaggcca aatacttctc ttctcctaga atggtaacgg aatattctct ggttaataga     480

tccctttcca gaagctgttc cacaaacgac atgccaacca ttccaaagcc caccaggaca     540

atttgctttg gcggagtggc caatccgttg tctgttattg tcattgtaag tgtgtagtca     600

agcaattgca aaacgttgac caggatgtcc actcgggtgg cctcggggtt tttcgaaccg     660

aactgataag atagtgcccc attttcggga cgaaagacgc cgatccgaga taagacccct     720

cggaacatgg tgataagtaa tcctcgcaaa tagattggac agtttgacaa tgattagagt     780

tttggagaat atatagacgt tatcagtaca tgggggggga tagttggcat tgactattg      839


<210>  18
<211>  420
<212>  DNA
<213>  Artificial Sequence

<220>
<223>  /note="promoter element AYNR1´-2, SEQ ID NO 18"

<400>  18
gttgtaggcc aaatacttct cttctcctag aatggtaacg gaatattctc tggttaatag      60

atccctttcc agaagctgtt ccacaaacga catgccaacc attccaaagc ccaccaggac     120

aatttgcttt ggcggagtgg ccaatccgtt gtctgttatt gtcattgtaa gtgtgtagtc     180

aagcaattgc aaaacgttga ccaggatgtc cactcgggtg gcctcggggt ttttcgaacc 240

gaactgataa gatagtgccc cattttcggg acgaaagacg ccgatccgag ataagacccc 300

tcggaacatg gtgataagta atcctcgcaa atagattgga cagtttgaca atgattagag 360

ttttggagaa tatatagacg ttatcagtac atgggggggg atagttggca ttgactattg 420

## Description

The present invention relates to a genetically manipulated yeast which is suitable for use in the production of n-butanol by fermentation of an organic substrate, and to a process for producing n-butanol using the genetically manipulated yeast in a fermentation process, which is preferably semi-anaerobic or anaerobic. As a substrate, biologic waste, e.g. excrements from animals, household sewage, waste containing protein, fats, alkanes, especially n-alkanes, alcohols, especially ethanol, starch, lignocellulose and/or tannines, preferably in an aqueous composition can be used. Preferably, the substrate contains sugar, e.g. a pentose and/or a hexose sugar which is comprised of monomeric, oligomeric or polymeric sugar or sugar alcohol moieties, e.g. starch, and/or alcohol, e.g. ethanol.

### State of the art

US 2007/0092957 Al describes micro-organisms, especially bacteria which are genetically manipulated to contain an acetolactate synthase, catalyzing the conversion of pyruvate to acetolactate, an acetohydroxy acid isomerase reductase for conversion of acetolactate to 2,3-dihydroxy isovalerate, an acetohydroxy acid dehydratase for conversion of 2,3-dihydroxy isovalerate to α-ketoisovalerate, a branched chain - ketoacid decarboxylase for conversion of α-ketoisovalerate to isobutyraldehyde, and a branched chain - alcohol dehydrogenase for conversion of isobutyraldehyde to isobutanol.

Ezeji et al in Current Opinion in Biotechnology 220 - 227 (2007) describe the fermentation of butanol and ethanol by Clostridium acetobutylicum, including the genetic manipulation of the bacterium by overexpression of alcohol / aldehyde - dehydrogenase and downregulation of CoA-transferase, both resulting in a decrease in butanol production.

WO 2008/097064 Al describes Saccharomyces cerevisiae containing the Coenzyme A - transferase encoding gene of Clostridium for butanol production.

WO 2009/103533 Al describes the genetic manipulation of Saccharomyces cerevisiae by overexpression of enzymes catalyzing the conversion steps from pyruvate to acetolactate, to 2,3-dihydroxyisovalerate, to 2-ketoisovalerate, to isobutyraldehyde, for the production of isobutanol.

WO 2009/013158 describes Saccharomyces cerevisiae for producing butanol which is genetically manipulated to contain a heterologous gene encoding an enzyme for conversion of acetyl-CoA to acetoacetyl-CoA, e.g. an acetyl-CoA acetyl transferase or thiolase, an enzyme converting acetoacetyl-CoA to 3-hydroxy butyryl-CoA, e.g. 3-hydroxy butyryl-CoA dehydrogenase, an enzyme converting 3-hydroxy butyryl-CoA to crotonyl-CoA, e.g. 3-hydroxy butyryl-CoA dehydratase, an enzyme converting crotonyl-CoA to butyryl-CoA, e.g. butyryl-CoA dehydrogenase, an enzyme converting butyryl-CoA to butyraldehyde, e.g. alcohol / aldehyde dehydrogenase, and an enzyme for converting butyraldehyde to n-butanol, e.g. an NAD(P)H - dependent butanol dehydrogenase, e.g. obtained from Clostridium.

### Objects of the invention

It is an object of the invention to provide for an alternative non-conventional yeast with high thermo- and osmotolerance, wide substrate spectrum and excellent growth behaviour which is capable of producing butanol by fermentation, especially at higher concentrations and/or with a lower concentration of by-products than prior art micro-organisms, and to provide a production process for n-butanol using the yeast.

### General description of the invention

The invention achieves these objects by providing a non-conventional, non-pathogenic yeast, and a process using the yeast, for producing n-butanol according to the claims, especially a yeast of the genus Arxula, especially Arxula adeninivorans, which has been genetically manipulated to contain a set of genes comprising or consisting of the genes encoding the pathway enzymes for the conversion of acetyl-CoA to butyryl-CoA from a heterologous micro-organism, especially from Clostridium, preferably from Clostridium acetobutylicum, e.g. genes encoding an acetyl-CoA-acetyl transferase, a β-hydroxybutyryl-CoA dehydrogenase, a crotonase , and a butyryl-CoA dehydrogenase, and optionally a butyryl aldehyde dehydrogenase.

In embodiments in which the yeast of the invention is of the genus Arxula, especially Arxula adeninivorans, the heterologous genes introduced into the yeast can consist of genes encoding the pathway enzymes for the conversion of acetyl-CoA to butyryl-CoA. The reason for the optional genetic manipulation of the yeast by introduction of heterologous genes consisting of those genes encoding the pathway enzymes for the conversion of acetyl-CoA to butyryl-CoA is, as has been discovered during the preparation of the present invention that Arxula contains an endogenous butyraldehyde dehydrogenase and an endogenous butanol dehydrogenase, which catalyzes the conversion of butyryl-CoA to n-butanol.

The yeast of the invention, and the process of the invention using the yeast are characterized in that the yeast in addition to being genetically manipulated by introduction of heterologous genes encoding the metabolic pathway enzymes for conversion of acetyl-CoA to butyryl-CoA, the yeast's metabolic pathway to or of the peroxisomal β-oxidation is impaired, preferably inactivated. This impairment or inactivation of the metabolic pathway to β-oxidation, e.g. the yeast can be genetically manipulated by impairment and/or inactivation of the gene encoding the peroxisomal acyl-carnitine permease.

Further alternatively or additionally, the yeast can be genetically manipulated by impairment and/or inactivation of the gene encoding the peroxisomal acyl-carnitine transferase which catalyzes the transfer of the butyryl moiety from butyryl-carnitine to peroxisomal butyryl-CoA.

Further optionally, additionally or alternatively, the yeast can be genetically manipulated by impairment and/or inactivation of the gene encoding the peroxisomal acyl-CoA-oxidase which catalyzes the oxidation of butyryl-CoA. The oxidation of butyryl-CoA within the peroxisome is currently regarded as the metabolic step towards β-oxidation of butyryl-CoA, and therefore impairment or inactivation of at least one of the genes encoding an enzyme that participates in the pathway from cytosolic butyryl-CoA towards β-oxidation is regarded as the cause for the increase in n-butanol yield in the fermentation.

Additionally, the yeast of the invention can be genetically manipulated for impairment or interruption of the pathway of glycerol synthesis, e.g. by impairing or inactivating at least one of the genes encoding an enzyme that participates in the synthesis of glycerol from dihydroxyacetone-3-phosphate, e.g. by impairing, preferably by inactivating the glycerol-3-phosphate dehydrogenase gene.

Generally, impairment or inactivation of a metabolic enzyme can be by impairment or inactivation of the gene encoding the enzyme, preferably by mutation of the gene, e.g. interruption, e.g. by insertion of one or more nucleotides, or partial or complete deletion of the gene encoding the enzyme.

The genetically manipulated yeast of the invention has the advantage of being tolerant to n-butanol, allowing comparatively high concentrations of n-butanol being produced in the fermentation medium. Further, the impairment, i.e. a reduction in flux of the metabolic pathway from cytosolic butyryl-CoA to butyryl carnitine, and further the impairment of the metabolic flux to peroxisomal butyryl carnitine, butyryl-CoA followed by β-oxidation, i.e. from butyryl-CoA to acetyl-CoA, increases the flux from butyryl-CoA originating from crotonyl-CoA towards butyraldehyde, respectively, and results in an increase in n-butanol production.

For impairment or inactivation of the transfer of butyryl-CoA or of butyryl-carnitine, respectively, to the peroxisome, it is preferred to delete the gene encoding peroxisomal acyl-carnitine transferase and/or to delete the gene encoding the peroxisomal acyl-carnitine permease specific for acyl-carnitine and butyryl-carnitine.

For impairment or inactivation of β-oxidation, it is preferred to inactivate the gene encoding the acyl-CoA oxidase. Generally, inactivation of an endogenous metabolic enzyme is by inactivation of the endogenous gene encoding the metabolic enzyme, preferably by mutation of the gene, which mutation is e.g. an insertion of at least one nucleotide into the gene, preferably the at least partial or the complete deletion of the gene.

For the optional additional impairment or inactivation of the conversion of dihydroxyaceton-3-phosphate to glycerol, it is preferred to delete the gene encoding glycerol-3-phosphate dehydrogenase. Generally, CoA designates coenzyme A.

The genetic manipulation of the yeast can be achieved by impairment or interruption of the yeast's metabolic pathway to peroxisomal ß-oxidation or of the peroxisomal ß-oxidation and results in an increased yield of n-butanol production. This genetic manipulation can e.g. be a mutation which inactivates gene function, e.g. a mutation, preferably a deletion of at least one gene of this metabolic pathway.

In a preferred embodiment, the yeast is Arxula, and the genetic manipulation, e.g. introduction of the metabolic pathway enzymes catalyzing the conversion of acetyl-CoA to n-butanol, consists of the introduction of the genes encoding the pathway enzymes catalyzing the conversion of acetyl-CoA to butyryl-CoA, i.e. of the genes encoding an acetyl-CoA-acetyl transferase, a β-hydroxybutyryl-CoA dehydrogenase, a crotonase and a butyryl-CoA dehydrogenase from Clostridium. The introduction of genes encoding these enzymes has the advantage of utilising the endogenous pathway enzymes catalyzing the conversion of butyryl-CoA to n-butanol, i.e. the butyraldehyde dehydrogenase and butanol-dehydrogenase encoded by Arxula.

Optionally, in a yeast of the invention, both the heterologous genes introduced into the yeast and, if applicable, the endogenous gene encoding butyraldehyde dehydrogenase and the endogenous gene encoding butanol dehydrogenase have a promoter with increased activity, e.g. a constitutive promoter with increased activity. Preferably, both the heterologous genes introduced into the yeast and, if applicable, the endogenous gene encoding butyraldehyde dehydrogenase and the endogenous gene encoding butanol dehydrogenase have a promoter with increased activity, preferably an inducible promoter, e.g. a nitrate and/or nitrite inducible promoter. A preferred promoter, which is inducible by nitrate and/or nitrite is selected from the group comprising the promoters of *AYNR1, AYNR1', AYNI1, AYNI1'* and *AYNT1, AYNT1',* which originate from the nitrate reductase gene (*AYNR1*), the nitrite reductase gene (*AYNI1*), and from the nitrate transporter gene (*AYNT1*) of Arxula adeninivorans, as well as truncated promoters of these.

The production process comprises the steps of
providing a genetically manipulated yeast of the invention,
providing a substrate in an aqueous composition in a fermentation vessel under control of pH and temperature,
preferably with controlled introduction of air, e.g. sparging with air, preferably controlled by the dissolved oxygen concentration, and with optional agitation, and
isolating or separating n-butanol, optionally in combination with acetone and/or ethanol, from the fermentation broth resulting from the cultivation of the yeast in the substrate composition.

Preferably, the fermentation process comprises a first aerobic phase in an aqueous medium composition containing substrate without inductor, e.g. without nitrate and without nitrite in the case of a yeast containing the butanol pathway enzyme encoding genes under the control of a nitrate and/or nitrite inducible promoter, e.g. containing monomeric or polymeric amino acids and/or ammonia as a nitrogen source, and a second semi-anaerobic or anaerobic phase which is commenced by addition of the inductor, e.g. nitrate and/or nitrite. The second phase of the fermentation process can be carried out as a fed-batch fermentation or as a continuous fermentation by addition of substrate. Preferably, n-butanol, ethanol and/or acetone are isolated continuously from the fermentation broth, e.g. by pervaporation through a membrane, under reduced pressure, i.e. under vacuum, by stripping using a gaseous or liquid stripping medium, or by a combination of these processes. Isolation of n-butanol, ethanol and/or acetone from the fermentation broth can occur in the fermentation vessel, or from a withdrawal pipe of the vessel with optional recirculation of the fermentation broth after isolation of n-butanol, ethanol and/or acetone.

The fermentation and cultivation process advantageously is carried out at a cultivation temperature of 10 to 70 °C, preferably 25 to 50 °C, using a genetically modified Arxula adeninivorans, because the elevated temperature allows an effective isolation of n-butanol, ethanol and/or acetone from the fermentation broth. The elevated cultivation temperature can be used, because the genetically modified Arxula is capable of growth and of butanol synthesis at these temperatures.

### Detailed description of the invention

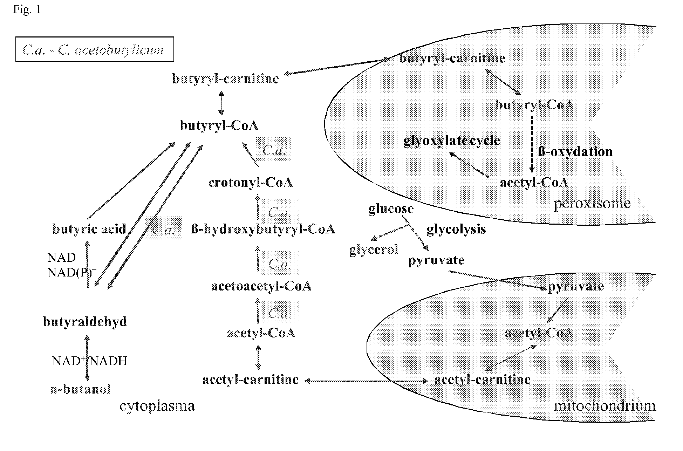

The invention is now described in greater detail with reference to the figures by way of examples. The figures schematically show in
- Figure 1: An overview of the metabolism as catalyzed by homologous and heterologous pathway enzymes expressed in the yeast which are essential for the butanol synthesis.

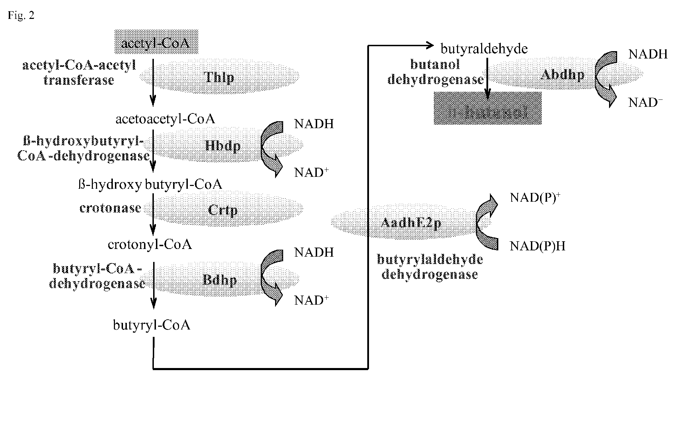

- Figure 2: Butanol metabolism from acetyl-CoA to butanol after insertion of the expression modules containing nucleic acid sequences encoding the Clostridium acetobutylicum enzymes acetyl-CoA-acetyl transferase (Thlp), ß-hydroxybutyryl-CoA-dehydrogenase (Hbdp), crotonase (Crtp), and butyryl-CoA-dehydrogenase (Bdhp), including the homologous genes encoding butyraldehyde dehydrogenase (AadhE2p) and butanol dehydrogenase (Abdhp) of Arxula adeninivorans.

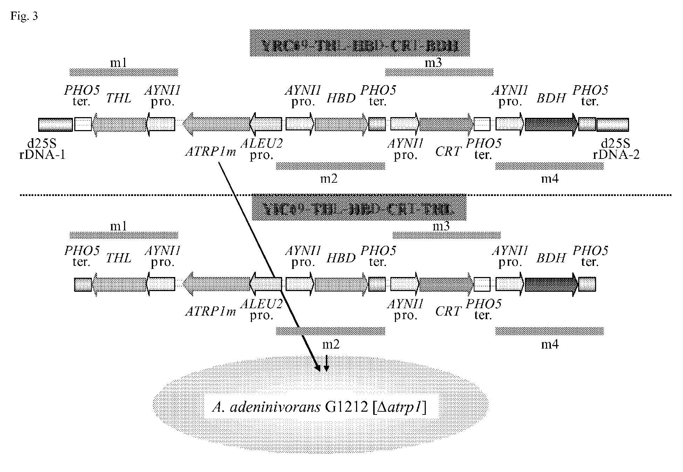

- Figure 3: Nucleic acid constructs YRC69-THL-HBD-CRT-BDH and YIC69-THL-HBD-CRT-BDH, each containing expression modules encoding the Clostridium acetobutylicum enzymes Thlp, Hbdp, Crtp, and Bdhp used for expression of these pathway enzymes participating in or constituting the metabolic pathway from acetyl-CoA to butyryl-CoA in Arxula adeninivorans (strain G1212).

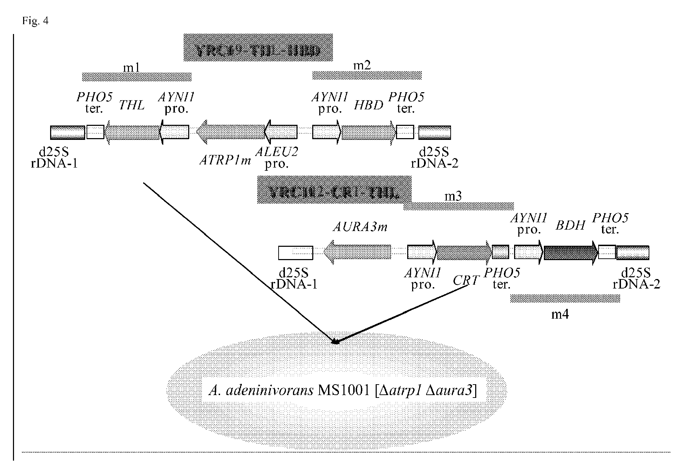

- Figure 4: Nucleic acid constructs YRC69-THL-HBD - YRC102-CRT-BDH and YIC69-THL-HBD - YIC102-CRT-BDH, each containing expression modules encoding the Clostridium acetobutylicum enzymes Thlp, Hbdp, and Crtp, Bdhp, respectively, for expression of these pathway enzymes participating in or constituting the metabolic pathway from acetyl-CoA to butyryl-CoA in Arxula adeninivorans (strain MS1001).

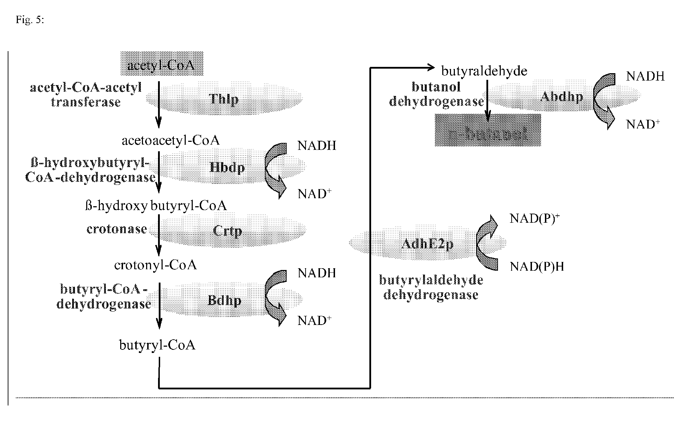

- Figure 5: An overview of butanol metabolism from acetyl-CoA to butanol after insertion of the expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p) including the homologous gene encoding butanol dehydrogenase (Abdhp) of Arxula adeninivorans.

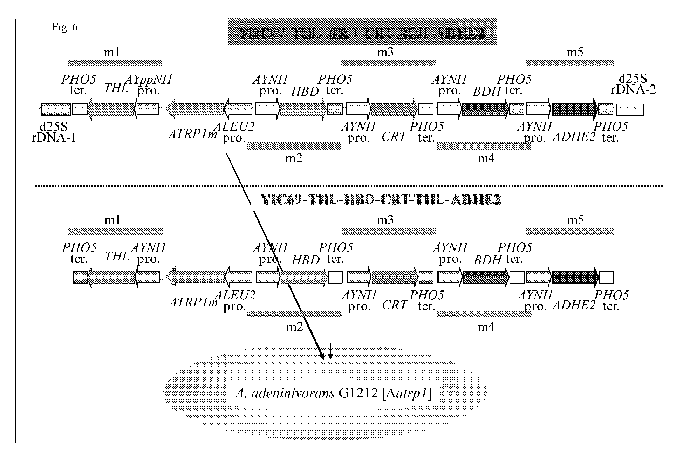

- Figure 6: Nucleic acid constructs YRC69-THL-HBD-CRT-BDH-ADHE2 and YIC69-THL-HBD-CRT-BDH-ADHE2, each containing expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p), respectively, for the metabolic pathway from acetyl-CoA to butyraldehyde in Arxula adeninivorans (strain G1212).

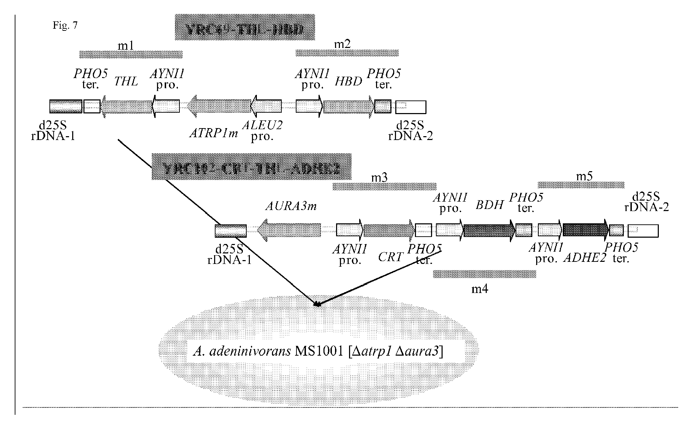

- Figure 7: Nucleic acid constructs YRC69-THL-HBD - YRC 102-CRT-BDH-ADHE2 and YIC69-THL-HBD - YIC102-CRT-BDH-ADHE2, containing expression modules encoding the Clostridium acetobutylicum enzymes acetyl-CoA-acetyl transferase (Thlp), ß-hydroxybutyryl-CoA-dehydrogenase (Hbdp), crotonase (Crtp), butyryl-CoA-dehydrogenase (Bdhp) and butyraldehyde dehydrogenase (AdhE2p), respectively, for constituting the metabolic pathway from acetyl-CoA to butyraldehyde in Arxula adeninivorans (strain MS1001).

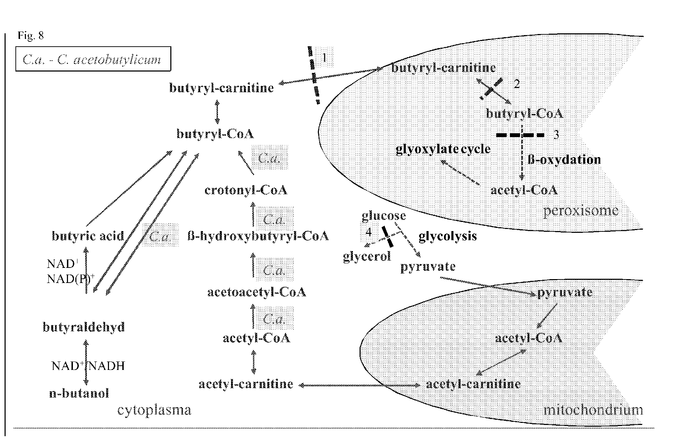

- Figure 8: An overview of the metabolism as catalyzed by homologous and heterologous pathway enzymes expressed in the yeast which are essential for butanol synthesis including inactivation of the peroxisomal acyl-carnitine permease 1 (Aacpp), the acyl-carnitine transferase 2 (Aactp), the acyl-CoA oxidase 3 (Aacop), and/or the glycerol-3-phosphate dehydrogenase 4 (Agpd1p) to improve the butanol level.

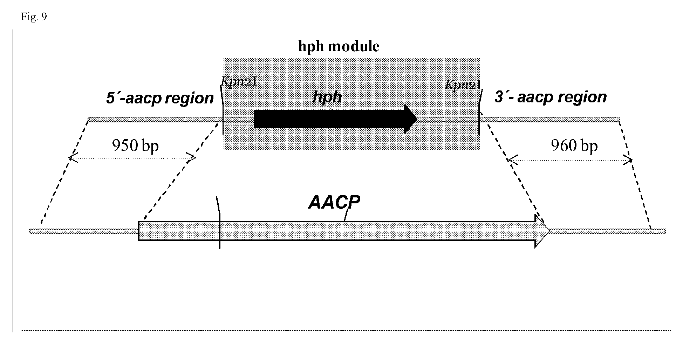

- Figure 9: Construction procedure for an Arxula adeninivorans aacp gene disruption mutant.

Preferably, the acetyl-CoA-acetyl transferase (Thlp) has the amino acid sequence of SEQ ID NO 1,
the β-hydroxy butyryl-CoA dehydrogenase (Hbdp) has the amino acid sequence of SEQ ID NO 2,
the crotonase (3-hydroxybutyryl-CoA dehydratase, Crtp) has the amino acid sequence of SEQ ID NO 3,
the butyryl-CoA dehydrogenase (Bdhp) has the amino acid sequence of SEQ ID NO 4. These sequences SEQ ID NO 1 to SEQ ID NO 4 originate from Clostridium.

Preferably, the butyraldehyde dehydrogenase (AadhE2p) and the butanol dehydrogenase (Abdhp) are the endogenous enzymes of Arxula.

As an alternative or in addition to the endogenous AadhE2p of Arxula, the heterologous butyraldehyde dehydrogenase (AdhE2) having SEQ ID NO 5 from Clostridium acetobutylicum can be expressed in the yeast of the invention.

More preferably, the amino acid sequences of acetyl-CoA- acetyl transferase (Thlp), of β-hydroxybutyryl-CoA dehydrogenase (Hbdp), of crotonase (Crtp), of butyryl-CoA dehydrogenase (Bdhp) and of butyraldehyde dehydrogenase (AdhE2p) are encoded by nucleic acid sequences which are codon-optimized for Arxula, e.g. by a coding sequence having a codon usage of the codons as preferred in Arxula for optimum translation. Preferably, the acetyl-CoA- acetyl transferase is encoded by codon-optimized SEQ ID NO 6, the β-hydroxy butyryl-CoA dehydrogenase is encoded by codon-optimized SEQ ID NO 7, the crotonase is encoded by codon-optimized SEQ ID NO 8, the butyryl-CoA dehydrogenase is encoded by codon-optimized SEQ ID NO 9, and the heterologous butyraldehyde dehydrogenase is encoded by codon-optimized SEQ ID NO 10. Generally, a coding sequence in contained in an expression module, wherein e.g. the coding sequence is functionally arranged in 3' to a promoter, and in 5' to a terminator.

The preferred promoters for regulating expression of the genes are independently selected from the group of promoters which are inducible by nitrate and/or nitrite, preferably from promoters of *AYNR1* (SEQ ID NO 11), *AYNR1'* (SEQ ID NO 12), *AYNI1* (SEQ ID NO 13), *AYNI1'* (SEQ ID NO 14) und *AYNT1* (SEQ ID NO 15), *AYNT1'* (SEQ ID NO 16) which originate from the nitrate reductase gene (*AYNR1*), nitrite reductase gene (*AYNI1*), and from the nitrate transporter gene (*AYNT1*) of Arxula adeninivorans, as well as truncated promoters of these, preferably a truncated variant of *AYNR1* termed *AYNR1-2* (SEQ ID NO 17) and/or a truncated variant of *AYNR1'* termed *AYNR1' -2* (SEQ ID NO 18).

### Example 1: Generation of a yeast which is genetically manipulated to contain coding

### sequences for the metabolic pathway to n-butanol

An Arxula adenininvorans strain having at least one auxotrophic marker was transformed with nucleic acid constructs containing a complementing gene as a selection marker. The Arxula adeninivorans contained an impaired metabolic pathway converting acyl-carnitine to peroxisomal acetyl-CoA. This yeast was obtained by interrupting the acyl-carnitine transferase gene, or alternatively one of the genes from the gene encoding the peroxisomal acyl-carnitine permease, the gene encoding the acyl-CoA oxidase, and the gene encoding the glycerol-3-phosphate dehydrogenase. Gene interruption was obtained by genetic manipulation of Arxula as described in Steinborn G, Wartmann T, Gellissen G, Kunze G (2007) Construction of an Arxula adeninivorans host-vector system based on trp1 complementation. J. Biotechnol. 127: 392-401.

According to a further preferred embodiment, wherein each sequence encoding an enzyme is contained in an expression module under the control of a nitrate and/or nitrite inducible promoter with a terminator element represented by the PHO5 terminator. As depicted, the expression modules encoding acetyl-CoA- acetyl transferase (Thlp, SEQ ID NO 1), the β-hydroxy butyryl-CoA dehydrogenase (Hbdp, SEQ ID NO 2), the crotonase (Crtp, SEQ ID NO 3), the butyryl-CoA dehydrogenase (Bdhp, SEQ ID NO 4) and butyraldehyde dehydrogenase (AdhE2p, SEQ ID NO 5) can be contained on one common nucleic acid construct. Optionally, the butyraldehyde dehydrogenase gene of Arxula (AadhE2), and/or the butanol dehydrogenase gene of Arxula (ABDH) in addition to the genomic gene copy is contained on the nucleic acid construct for genetically manipulating the yeast, especially for integrating an expression module having an inducible promoter, as exemplified by the nitrate and/or nitrite inducible promoter *AYNI1* (SEQ ID NO 13) for each coding sequence, for simultaneous and increased induction of the enzyme.

The Xplor^{®}2 transformation/expression platform was used for integration of the nucleic acid constructs into the Arxula adeninivorans genomic DNA (as e.g. described by Böer E, Piontek M, Kunze G (2009) Xplor®2 - an optimized transformation/expression system for recombinant protein production in the yeast A. adeninivorans. Appl Microbiol Biotechnol 84:583-594). The nucleic acid constructs are Yeast rDNA Integrative Expression Cassettes (YRCs), that contain homologous DNA sections at both termini, which are represented by the exemplary 25S rDNA sections d25S rDNA-1 and d25S rDNA-2 (YRC). The embodiments of nucleic acid constructs which are Yeast Integrative Expression Cassettes (YIC) do not contain the terminal homologous rDNA sections. Both the YRC and YIC contain a selection marker module, e.g. an expression cassette containing a gene for complementing an auxotrophy of the recipient yeast, as shown by the exemplary complementing gene ATRP1m serving as a selection marker under the control of the promoter ALEU2 which complements the tryptophan auxotrophy of a recipient yeast, e.g. Arxula adeninivorans G1212 [aleu2 Δatrp1::ALEU2] or MS1001 [aleu2 Δatrp1::ALEU2 aleu2 Δaura3::ALEU2]. In addition, the expression modules encoding Thlp, Hbdp, Crtp, Bdhp, and alternatively AdhE2p are localised on YRC and YIC (Figs. 3 and 6).

Figures 4 and 7 show an alternative cloning procedure for generating a genetically manipulated yeast of the invention. In this procedure, the expression modules encoding the pathway enzymes are contained on two separate YRC's, each of which has a complementing gene as a selection marker, and the recipient yeast Arxula adeninivorans MS1001 [aleu2 Δatrp1::ALEU2 aleu2 Δaura3::ALEU2] has the respective auxotrophy which is complemented by the respective selection marker. The first YRC (YRC69-THL-HBD) contains the expression modules encoding Thlp and Hbdp, each under the control of a nitrate and/or nitrite inducible promoter, and comprises the selection marker ATRP1m encoding sequence for complementing the atrp1 auxotrophy of the recipient Arxula adeninivorans MS1001 [aleu2 Δatrp1::ALEU2 aleu2 Δaura3::ALEU2], and the second YRC (YRC102-CTR-THL) contains the expression modules for CRT, BDH, and optionally for ADHE2, which can be an alternative to or an addition to the endogenous gene of Arxula, as well as the selection marker AURA3m for complementing the aura3 auxotrophy of the recipient yeast Arxula adeninivorans MS1001 [aleu2 Δatrp1::ALEU2 aleu2 Δaura3::ALEU2].

As shown for the YRC's of Figures 3,4,6,7, the nucleic acid constructs can contain up to five expression modules for the metabolic pathway enzymes for n-butanol plus the respective selection marker. These nucleic acid constructs are flanked at both termini by homologous sections for homologous recombination and integration, e.g. d25S rDNA sections d25S rDNA -1 and d25S rDNA -2. In contrast YIC's, prefer the non-homologous recombination for their integration in the genomic DNA.

Following transformation of the auxotrophic Arxula strains G1212 and/or MS1001 according to standard procedures, selection of transformants was by cultivation on yeast minimal medium as described by Tanaka A, Ohnishi N, Fukui S (1967) Studies on the formation of vitamins and their function in hydrocarbon fermentation. Production of vitamin B6 by Candida albicans in hydrocarbon medium. J Ferment Technol 45:617-623.

Figures 2, 5 and 8 give an overview of the metabolic pathway provided by the yeast and the heterologous genes with the impairments of the metabolism.

### Example 2: Arxula adeninivorans strain optimization by gene disruption

Impairment of the metabolic flux from butanol synthesis towards peroxisomal catabolism is achieved by inactivating the peroxisomal acyl-carnitine permease 1 (Aacpp), the acyl-carnitine transferase 2 (Aactp), the acyl-CoA oxidase 3 (Aacop), and/or the glycerol-3-phosphate dehydrogenase 4 (Agpd1p). For this purpose, the Arxula adeninivorans genes AACP, AACT, AACO and/or AGPD1 were mutated by gene disruption. Since Arxula adeninivorans MS1001 is a hygromycin B sensitive strain, all gene disruption mutants were initially selected as hygromycin B resistant transformants by using an expression cassette containing the TEF1 promoter - hph gene (from E. coli) - PHO5 terminator which was flanked by ca. 950 bp Δacpp, Δaact, Δaaco and Δagpd1 fragments. E.g., the Δaacp fragment with 950 bp 5'-aacp region - hph expression module - 960 bp 3'-aacp region was amplified by PCR and used to transform Arxula adeninivorans MS1001 for sequential disruption of the chromosomal AACP copy. For selection of the respective gene disruption mutants, the transformants were firstly selected for hygromycin resistance, and secondly selected for aacp deletion by amplification of the inserted 5'-aacp region - hph expression cassette - 3'-aacp region and Southern hybridization with the AACP fragment as labelled probe (Fig. 4).

Δaact, Δaaco and Δagpd1 gene disruption mutants were constructed using the same strategy.

### Example 3: Production of n-butanol from a starch substrate by fermentation

An Arxula strain that was obtained by the cloning procedure according to Example 1 was cultivated in an aqueous composition of starch, containing ammonia as a buffering substance in the absence of nitrate and absence of nitrite at 25-48°C under aerobic conditions in an agitated 3 L fermenter. Subsequent to an initial cultivation phase in the absence of inductor, nitrate was added and an aqueous starch composition containing 10-50 g/L starch was added for fed-batch fermentation. Analysis of the fermentation broth by gas chromatography with mass spectrometric detection showed production of 20 g/L n-butanol. N-butanol was separated from the fermentation broth by standard methods.

## Claims

1. Genetically manipulated yeast containing heterologous nucleic acid sequences encoding pathway enzymes for the synthesis of n-butanol from a substrate, **characterized in that** the yeast is of the genus Arxula, and the heterologous pathway enzymes comprise an acetyl-CoA-acetyl transferase (Thlp), a β-hydroxy butyryl-CoA dehydrogenase (Hbdp), a crotonase (Crtp), and a butyryl-CoA dehydrogenase (Bdhp), and each encoding nucleic acid sequence is arranged in an expression module, in which yeast the metabolic pathway from acyl-carnitine to β-oxidation is impaired or interrupted.

2. Genetically manipulated yeast according to claim 1, **characterized in that** the heterologous pathway enzymes consist of an acetyl-CoA-acetyl transferase (Thlp), a β-hydroxy butyryl-CoA dehydrogenase (Hbdp), a crotonase (Crtp), and a butyryl-CoA dehydrogenase (Bdhp).

3. Genetically manipulated yeast according to claim 1 or 2, **characterized in that** each expression module the heterologous nucleic acid sequences encoding pathway enzymes for the synthesis of butanol are each independently arranged under the control of a promoter which is inducible by nitrate and/or nitrite, a heat inducible promoter, and/or a promoter inducible by the substrate

4. Genetically manipulated yeast according to one of the preceding claims, **characterized in that** the yeast contains a heterologous nucleic acid sequence encoding butyraldehyde dehydrogenase (AdhE2p).

5. Genetically manipulated yeast according to one of the preceding claims, **characterized in that** each expression module containing a nucleic acid sequence encoding a pathway enzyme for the synthesis of n-butanol contains the same promoter.

6. Genetically manipulated yeast according to one of the preceding claims, **characterized in that** the metabolic pathway from acyl-carnitine to β-oxidation is impaired or interrupted by mutation of at least one of the endogenous genes encoding at least one of the peroxisomal acyl-carnitine transferase, the peroxisomal acyl-carnitine permease and/or the acyl-CoA oxidase.

7. Genetically manipulated yeast according to one of the preceding claims, **characterized in that** the metabolic pathway from dihydroxyacteone phosphate to glycerol is impaired or interrupted by mutation of the gene encoding the glycerol-3-phosphate dehydrogenase.

8. Genetically manipulated yeast according to one of the preceding claims, **characterized in that** the acetyl-CoA-acetyl transferase has the amino acid sequence of SEQ ID NO 1, the β-hydroxy butyryl-CoA dehydrogenase has the amino acid sequence of SEQ ID NO 2, the crotonase has the amino acid sequence of SEQ ID NO 3, the butyryl-CoA dehydrogenase has the amino acid sequence of SEQ ID NO 4, and the optional butyraldehyde dehydrogenase (AdhE2p) has the amino acid sequence of SEQ ID NO 5.

9. Genetically manipulated yeast according to one of the preceding claims, **characterized in that** the butyraldehyde dehydrogenase and the butanol dehydrogenase are endogenous enzymes.

10. Genetically manipulated yeast according to one of the preceding claims, **characterized in that** the acetyl-CoA-acetyl transferase is encoded by SEQ ID NO 6, the β-hydroxy butyryl-CoA dehydrogenase is encoded by SEQ ID NO 7, the crotonase is encoded by SEQ ID NO 8, the butyryl-CoA dehydrogenase is encoded by SEQ ID NO 9, and the optional additional butyraldehyde dehydrogenase is encoded by SEQ ID NO 10.

11. Process for producing n-butanol from a substrate by cultivation of a yeast in an aqueous fermentation broth containing the substrate, **characterized in that** the yeast is a yeast according to one of the preceding claims, and n-butanol is isolated from the fermentation broth.

12. Process according to claim 11, **characterized in that** the cultivation and fermentation process is carried out at a temperature between 10 to 70°C, preferably between 25 to 50°C.

13. Process according to one of claims 11 to 12, **characterized in that** the cultivation comprises a first aerobic phase in a fermentation broth in the absence of inductor, and a subsequent second phase is commenced by addition of inductor.

14. Process according to one of claims 11 to 13, **characterized in that** the cultivation and fermentation processes are carried out as continuous processes.

15. Process according to one of claims 11 to 14, **characterized in that** the substrate is selected from aqueous mixtures containing excrements from animals, household sewage, waste containing protein, fats, alkanes, n-alkanes, alcohols, ethanol, starch, lignocelluloses, tannins, pentose and/or hexose sugars which are comprised of monomeric, oligomeric or polymeric sugar or sugar alcohol moieties, and mixtures thereof.
